# EUROPEAN PATENT SPECIFICATION

(11) **EP 1 951 334 B1**
(45) Date of publication and mention of the grant of the patent: **14.10.2015**
(21) Application number: 06804880.0
(22) Date of filing: 21.11.2006
(51) Int. Cl.: A61M 1/06, A61N 1/36

(54) **BREASTPUMP**
BRUSTPUMPE
TIRE-LAIT

(30) Priority: 25.11.2005 US 739675 P
(43) Date of publication of application: 06.08.2008
(73) Proprietor: Medela Holding AG, 6340 Baar (CH)
(72) Inventor: HARTMANN, Peter Edwin, Gooseberry Hill, Wa 6076 (AU); GEDDES, Donna Tracy, Dianella, WA 6059 (AU); KENT, Jacqueline Coral, Nedlands, WA 6009 (AU); CREGAN, Mark, Derek, Clarkson, WA 6030 (AU)
(74) Representative: Clerc, Natalia
(86) International application number: PCT/CH2006/000653
(87) International publication number: WO 2007/059642

(56) References cited:
- WO-A-00/07658
- US-A- 4 243 043
- US-A1- 2003 065 277
- US-A1- 2005 059 928

## Description

### Field of the invention

This invention relates to a breast pump for expressing mother's milk according to the preamble of claim 1.

### Background of the invention

Breastpumps for expressing mother's milk to feed an infant are well known in the state of the art. They generally comprise a hood or breastshield that fits over the mother's breast, a vacuum or low pressure pump connected to the shield for generating an intermittent vacuum or negative pressure within the shield and a receptacle for the expressed milk. The intermittent suction action of the vacuum within the shield serves to pull on and massage the breast and thereby extract milk in an action reminiscent of suckling. There are known breastpumps which can be used in both single and double modes of operation. This means one single breast or both breasts can be expressed at the same time.

WO 01/47577 discloses a breastpump which can be programmed to generate a plurality of differing milk expression sequences. The programs include a suction sequence for a sore nipple condition, a suction method for increased milk production, an improved suction method in general and a method for nipple stimulation. This different programs use the variation of suction frequencies and vacuum power applied. This breastpump is a highly appreciated device used in hospitals as well as at home helping the mother to express the milk in a most "babylike" manner.

US 2005/059928 discloses a breastpump comprising a breastshield with electrodes arranged in the funnel shaped portion of this breastshield. These electrodes are capable of sensing changes in the breast.

US 2003/0065277 discloses an aspirator for obtaining a fluid sample from the human breast.

Leaving now the technical field of breastpumps and entering the field of pain control treatment, a device called TENS (transcutaneous electrical nerve stimulation) is known. The TENS generates electrical impulses which are applied to the nerves by electrodes placed on or near the painful site. A TENS device treats many kinds of pain. It can lessen acute pain, like after surgery or an accident. Chronic pain, like arthritis, back or muscle pain may also be lessened with TENS. TENS has also successfully been used by pregnant women to treat labour pain. At the onset of labour, electrodes in the shape of pads are applied to the back of the mother and the TENS unit send electrical impulses to the pads. When the mother experiences a contraction she can press a booster button for an extra surge of power.

### Summary of the invention

It is an object of the invention to provide an improved breastpump which optimizes the stimulation of the mother's breast.

This object is achieved with a breastpump having the features of claim 1.

The inventive breastpump comprises a breastshield having a portion within which a woman's breast is received for the expression of milk, a source of iow pressure in communication with said breastshield and means for transcutaneous electrical nerve stimulation (TENS) of the woman. The electrical impulses can be applied on the woman's breast itself or on any other appropriate place on the women which help to increase or stimulate the milk ejection.

By applying electrical impulses the nerves in the breast are stimulated and the milk ejection reflex is activated. The TENS can activate the medial nerves, the lateral nerves, the afferent nerves or combinations thereof. Furthermore the electrical impulses are preferably applied before the vacuum or low pressure is applied, i.e. before the milk extraction starts. However, it is also possible to apply the electrical impulses in addition or only during extraction of the milk.

In a preferred embodiment the TENS unit which generates the impulses is connected to the breast pump unit comprising the vacuum source. In another preferred embodiment, the TENS unit is an integral part of this breast pump unit.

The electrodes of the TENS are attached or can be attached to the breast-shield. In another example, the electrodes are separate tools, especially pads, which can be applied at the mother's breast at a convenient place thereof. Preferably the pads are formed in such a shape to exclude the nipple of the breast. In other embodiments the nipples are stimulated as well. Two pads per breast are preferred, however one or three or more pads per breast can be used too.

According to an example, the electrodes are located such as to stimulate the nerve at the side of the rib cage and/or the anterior cutaneous nerve and/or the lateral cutaneous nerve. The electrodes can be located between 1 o'clock and 11 o'clock, preferably in the region between 1 o'clock and 4 o'clock and/or in the region between 8 o'clock and 11 o'clock. In this case the electrodes are inbuilt into or attached to the breast shield, even more preferably arranged in or attached to a funnel shaped portion of the breast shield.

It is preferred, if the electrodes are not located on the nipple and/or too close to it, as this may have negative impact on the stimulation of expression.

According to a further preferred embodiment of the breast pump the TENS unit provides continuous and/or sweeped and/or pulsed stimulation. Generally one can say, that TENS stimulation should be carried out with a current in the range below 200 µA, preferably below 100 µA, even more preferably in the range of 10-30 µA.

According to a further preferred embodiment, the TENS unit provides pulsed stimulation in a frequency range of 0-250 Hz, preferably in a frequency range of 10-250 Hz, wherein the pulse length is preferably in the range of 10-1000 µs, even more preferably in the range of 50-200 µs.

The breastpump can comprise a unit as described in the above mentioned WO 01/47577. However, the TENS stimulation is considered to be a substitute for the stimulation which uses different sequences of the applied vacuum. The TENS unit can also be used with other breastpumps. Motor driven breastpumps are preferred, but it also works with manually operated breastpumps.

The inventive breastpump can not only be used by mother's having given birth to a child but also by mother's of adopted children wanting to breastfeed the adopted infant.

It should further be noted that it is possible to program the TENS unit such as to provide interferential current therapy (IFC).

The present disclosure furthermore relates to a specific breast shield for the combined breast pump assisted extraction of milk from a mother and for TENS stimulation.

Such a breastshield, in particular for use in a breast pump as described above, is characterised in that electrodes are inbuilt into or attached to the breastshield, wherein preferably the breastshield comprises a funnel shaped portion and wherein the electrodes are arranged in or attached to this funnel shaped portion. Preferably in such a breast shield the electrodes are located such as to stimulate the nerve at the side of the rib cage and/or the anterior cutaneous nerve and/or the lateral cutaneous nerve.

Correspondingly therefore, the breast shield is characterised in that the electrodes are located between 1 o'clock and 11 o'clock, preferably in the region between 1 o'clock and 4 o'clock and/or in the region between 8 o'clock and 11 o'clock.

Furthermore the present disclosure relates generally to the use of TENS with stimulation at the mother's breast and/or close to the mother's breast for the stimulation of milk expression and/or for the treatment of mastitis and/or for the treatment of blocked ducts and/or for the treatment of engorgement.

Further preferred embodiments of the invention are described in the dependent claims.

### Brief description of the drawings

The present invention will be more clearly understood with reference to the following detailed description of preferred embodiments, taken in conjunction with the accompanying drawings, in which
- Figure 1: shows an embodiment of a breastpump according to the invention;
- Figure 2: shows an example of a breastpump; and
- Figure 3: schematic views onto a woman's right breast, a) position of electrodes for anterior cutaneous nerve; b) position of electrodes for lateral cutaneous nerve; c) position of electrodes in typical TENS fashion.

### Description of the preferred embodiments

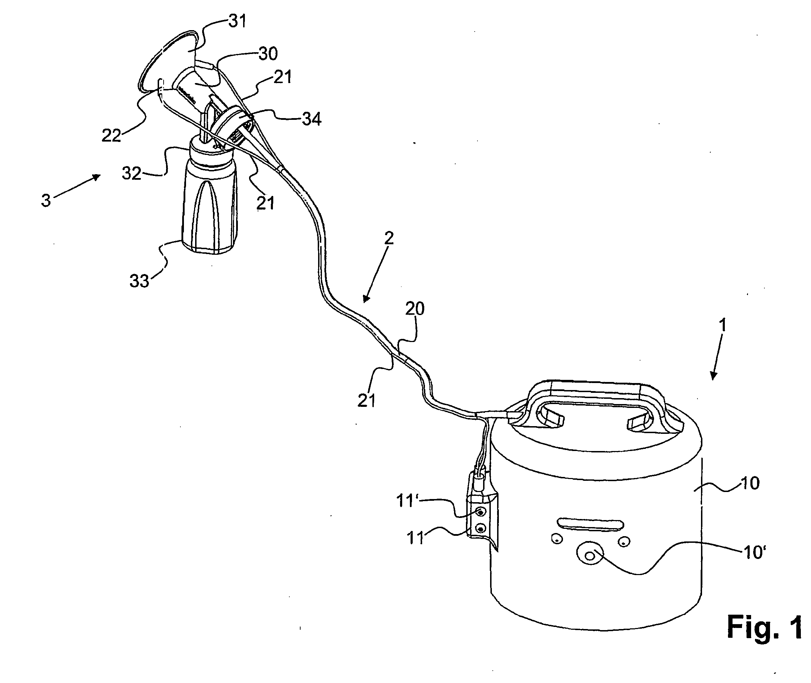

Figure 1 shows the inventive breastpump in a first embodiment. The breastpump comprises at least a breastpump unit 1, at least one breastshield 3 and a connection line 2.

The breastpump unit 1 comprises a casing 10 with a vacuum or low pressure source and operation means 10' for operating the vacuum source. For example the applied frequencies and vacuum level as well as the shape of the vacuum curves or sequences applied can be regulated either manually or by using programs. New sequencies can be stored using the operation means 10'. The operation means 10' can be knobs, input panels or the like. The shape of the casing can be different to the one shown, it can for example also be a soft casing and not a rigid one as shown in figure 1.

The breastpump unit 1 comprises further a TENS unit 11 for generating electrical impulses. In this embodiment the TENS unit 11 is attached to the casing 10 of the low pressure source. However it can also be integrated in the casing 10. The TENS unit 11 comprises operation means 11' as well. This means can be knobs, input panels or the like. They can also be coupled with the operation means of the vacuum source, for example they can be arranged on the same panel.

In a preferred embodiment, the breastpump unit 1 comprises control means which disables operation of the vacuum pump and the TENS at the same time. However in another preferred embodiment, such an operation is enabled.

The breastshield unit 3 comprises the classical breastshield with the funnel shaped front part 31 which is applied over the mother's nipple to the mother's breast. The funnel shaped front part 31 ends in a tubular extension 30 which divides in a first connection part 32 and a second connection part 34. The first connection part 32 can be attached, normally screwed on a collection bottle 33 or another appropriate collection item, such as a bag.

Electrodes 22 are arranged at, preferably inbuilt into, the funnel shaped front part 31 of the breastshield 3. We refer to WO 03/066133 which discloses a breastshield with integrated electrodes. The electrodes 22 penetrate the front part 31 material, which is usually a plastic, such as PP or TPE. In the embodiment shown there are two electrodes 22 fixed to the front part 31. However, only one or three or more electrodes can be used as well.

The connection line 2 comprises a suction line or vacuum tube 20 and one or more electrical wires 21. It can be a double lumen or the wire is simply fixed to the tube. Preferably the wire 21 is connected to said vacuum tube at least along a part of the length of the vacuum tube.

The vacuum tube 20 connects the vacuum source with the breastshield 3, i.e. with the second connection part 34 in order to apply a vacuum to the front part when this part is hold against a mother's breast. The wire 21 connects the TENS unit 10 with the electrodes 22 in order to transfer the electrical impulses to the breastshield, i.e. to the front part 31 thereof and to stimulate the mother's breast.

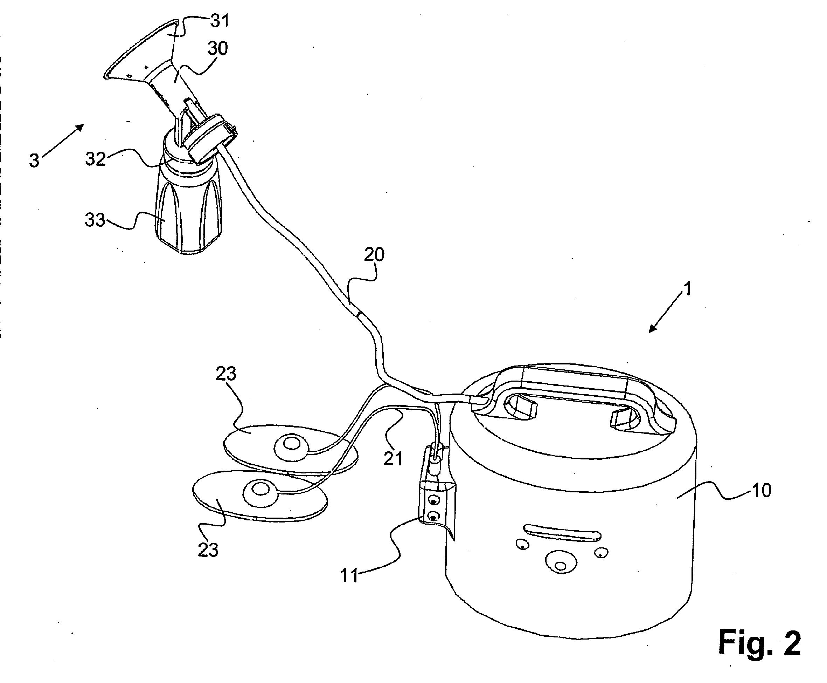

The example according to figure 2 is similar to the one described above. The main difference is that electrodes are not arranged within the breastshield 3 but are separate items. Therefore the connection line only comprises the vacuum tube 20 and the wire is a separate item as well, directly connecting the TENS unit 11 with the electrodes. In this example the electrodes are pads 23 which can be arranged freely on the mothers breast at any convenient place or at any other appropriate place on the woman. The shape of the pads 23 can be circular as shown or they can have any other appropriate shape which enables a good contact with the mother's skin.

In operation the electrodes are applied to the mother's breast, i.e. either the pads 23 are used or the breastshield 3 is put over the breast covering the nipples so that the electrodes contacts the skin of the breast. Then the TENS unit 11 is activated and electrical impulses are send to the mother's nerves, stimulating the breast and activating the milk ejection reflex. It is possible to adjust the frequencies and the power of the impulses to achieve optimum results. Furthermore, depending where the electrodes are applied on the breast, different kind of nerves, i.e. medial, lateral and afferent nerves, can be stimulated. It is therefore possible to use pads or breastshields with a special arrangement of electrodes thereon, wherein the TENS unit can activate special patterns of the electrodes as well as all of the electrodes all together. By activating different patterns of electrodes it is possible to stimulate only special nerves or combinations of these nerves.

When the stimulation is completed, low pressure can be applied to the breastshield and milk can be expressed directly or after a convenient break. However it is also possible to stimulate also during expression or to start with the electrical impulses and the appliance of vacuum at the same time.

The invention was described with one single breastshield only. However it is understood that it can also be used with double breast pumping, wherein only one or both breasts can be stimulated at the same time.

The invention enables a stimulation of the mother's breast in order to activate the milk ejection reflex.

It is known that nipple and areola are innervated by the 3rd , 4th or 5th intercostal nerves. The diameter of the lateral cutaneous nerve is greater than anterior cutaneous nerve (100 um vs 20 um). The lateral cutaneous nerve turns 90 degrees in midaxillary line and continues through glandular tissue toward posterior surface of the nipple. Deep course is most common though and superficial course is possible. The anterior cutaneous nerve travels in the subcutaneous tissues and terminates at the areola edge (8-11 o'clock in the left breast and 1-4 o'clock in the right breast).

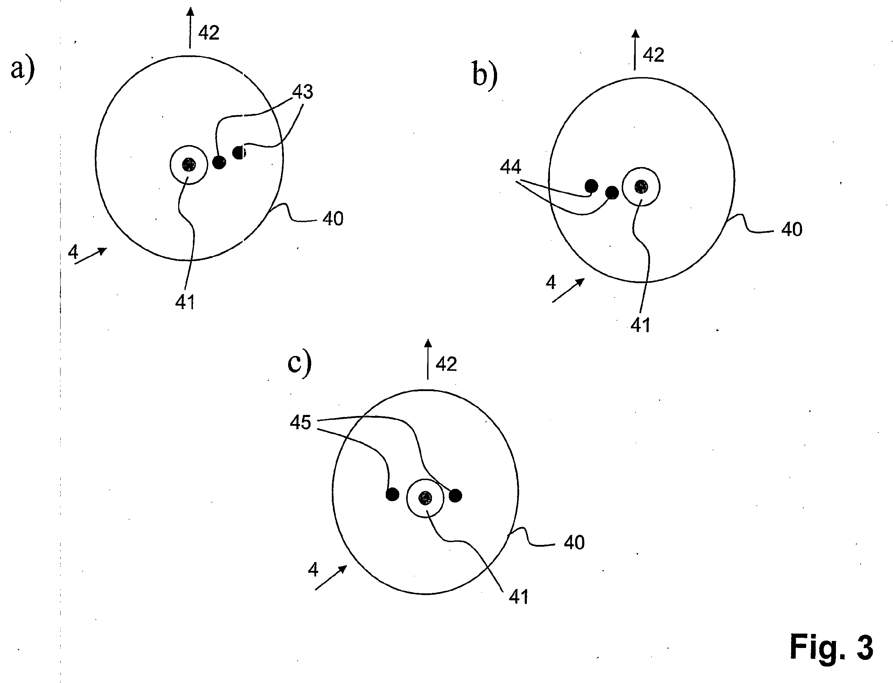

Correspondingly therefore preferably electrodes are located in medial and/or lateral positions. Since the anterior cutaneous nerve is most superficial this can be the most efficient nerve to stimulate. Electrodes can be placed anywhere from the base of the nipple to the periphery of the breast (8-11 o'clock in the left breast and 1-4 o'clock in the right breast). See diagrams as given in Figure 3.

The cathode and anode can be both placed along the course of the nerve. Similarly it is possible to use a situation where the cathode and anode are located on the opposite sides of the breast (medial and lateral). Continuous and pulsed delivery of TENS is possible in both cases. Different frequencies can be useful in the stimulation of milk expression as well as for the treatment of blocked ducts and mastitis or engorgement.

It should be noted that also interferential current therapy (IFC) can be used with the above arrangement.

IFT is based on the summation of 2 alternating current signals of slightly different frequency. The resultant current consists of cyclical modulation of amplitude, based on the difference in frequency between the 2 signals. When the signals are in phase they summate to an amplitude sufficient to stimulate, but no stimulation occurs if they are out of phase. The beat frequency of IFC is equal to the difference in the frequencies of the 2 signals. For example, the beat frequency and, hence, the stimulation rate of a dual channel IFC unit with signals set at 4200 and 4100 Hz is 100 Hz.

IFC can deliver higher currents than TENS. IFC can use 2, 4 or 6 applicators. IFC can be used in reduction of swelling or in women with engorgement.

### Experimental section:

### Specification of the TENS unit used for the experiments:

1. National; EW 431 w; Low-Frequency Muscle Stimulator
2. OSIM; Pro-Therapist; Model OS-205; Power Supply: S006P AC9V 1pc; Oscillation Frequency:1-250Hz; Power consumption: Max. about 45mA (Load 1k).

### Results:

1. Mother One - Her own home. Electrodes were placed either side of the nipple. Milk began to drip from the nipple within 10 seconds of stimulation with TENS 2. Mother One - Breastfeeding Centre; Left Breast; TENS - Electrodes on lateral side of Left breast

### Results Ultrasound scan for milk ejection (duct dilation in opposite breast)

| | |
|---|---|
| 1:03:33 | 3.0mm |
| 1:03:38 | 3.0mm |
| 1:03:50 | 4.0mm |
| 1:04:00 | 3.9mm |
| 17s to milk ejection | |

### Results Ultrasound scan for milk ejection (duct dilation in same breast).

| | |
|---|---|
| 1:49:35 | 3.5mm |
| 1:49:40 | 4.9mm |
| 1:49:48 | 5.0mm |
| 13s to milk ejection | |

### List of reference numbers

- 1: breastpump unit
- 10: vacuum pump
- 10': vacuum pump operation means
- 11: TENS unit
- 11': TENS unit operation means

- 2: connection line
- 20: vacuum tube
- 21: electrical wire
- 22: electrode
- 23: electrode pad

- 3: breastshield unit
- 30: tubular extension
- 31: funnel shaped part
- 32: first connection part
- 33: collection bottle
- 34: second connection part

- 4: right breast
- 40: periphery of 4
- 41: nipple/areola
- 42: upside
- 43: pair of electrodes for anterior cutaneous nerve
- 44: pair of electrodes for lateral cutaneous nerve
- 45: position of electrodes in typical TENS fashion

## Claims

1. A breastpump comprising a breastshield (30, 31) having a portion (31) within which a woman's breast is received for the expression of milk and a source of pressure (10) in communication with said breastshield (30, 31), **characterized in that** the breastpump has means (11, 21, 22, 23, 43, 44, 45) for transcutaneous electrical nerve stimulation (TENS) of the woman, wherein said means (11, 21, 22, 23, 43, 44, 45) for TENS comprises a TENS unit (11) for generating electrical impulses, electrodes (22, 23, 43, 44, 45) for applying said electrical impulses on said woman and an electrical wire (21) for sending said electrical impulses from the TENS unit (11) to the electrodes (22, 23, 43, 44, 45) and wherein the electrodes (22) are inbuilt into or attached to the breastshield (30, 31).

2. The breastpump according to claim 1, wherein the TENS unit (11) is attached to or inbuilt into said source of pressure (10).

3. The breastpump according to claim 1, wherein the breastshield (30, 31) comprises a funnel shaped portion (31) and wherein the electrodes (22) are arranged in or attached to this funnel shaped portion (31).

4. The breast pump according to any of the preceding claims, wherein the electrodes (22, 23, 43, 44, 45) are located on the breastshield between 1 o'clock and 11 o'clock, preferably in the region between 1 o'clock and 4 o'clock and/or in the region between 8 o'clock and 11 o'clock.

5. The breast pump according to any of the preceding claims, wherein the TENS unit (11) provides continuous and/or sweeped and/or pulsed stimulation, preferably with a current in the range below 200 µA, preferably below 100 µA, even more preferably in the range of 10-30 µA.

6. The breast pump according to claim 5, wherein the TENS unit (11) provides pulsed stimulation in a frequency range of 0-250 Hz, preferably in a frequency range of 10-250 Hz, wherein the pulse length is preferably in the range of 10-1000 µs, even more preferably in the range of 50-200 µs.

7. The breastpump according to any of the preceding claims, wherein the breastpump comprises a vacuum tube (20) for said communication between the source of pressure (10) and the breastshield (30, 31) and wherein the electrical wire (21) is connected to said vacuum tube (20) at least along a part of the length of the vacuum tube (20).

8. The breastpump according to any of the preceding claims, wherein it comprises control means (10', 11') to prevent operation of the vacuum source (10) and the TENS unit (11) at the same time.

9. The breast pump according to any of the preceding claims, wherein the TENS unit (11) is programmed to provide interferential current therapy (IFC).

## Patentansprüche

1. Brustpumpe, umfassend eine Brusthaube (30, 31) mit einem Bereich (31), in welchen eine weibliche Brust aufgenommen ist, um Milch abzupumpen, und mit einer Druckquelle (10), welche in kommunizierender Verbindung mit dieser Brusthaube (30, 31) steht, **dadurch gekennzeichnet, dass** die Brustpumpe Mittel (11, 21, 22, 23, 43, 44, 45) zur transkutanen elektrischen Nervenstimulation (TENS) der Frau aufweist, wobei diese Mittel (11, 21, 22, 23, 43, 44, 45) für TENS eine TENS Einheit (11) zur Erzeugung eines elektrischen Impulses, Elektroden (22, 23, 43, 44, 45) zum Anlegen des elektrischen Impulses an diese Frau und eine elektrische Leitung (21) zur Übermittlung dieses elektrischen Impulses von der TENS Einheit (11) zu den Elektroden (22, 23, 43, 44, 45) aufweist, und wobei die Elektroden (22) in die Brusthaube (30, 31) integriert oder an dieser angebracht sind.

2. Brustpumpe nach Anspruch 1, wobei die TENS Einheit (11) an der Druckquelle (10) angebracht oder in diese integriert ist.

3. Brustpumpe nach Anspruch 1, wobei die Brusthaube (30, 31) einen trichterförmigen Bereich (31) aufweist und wobei die Elektroden (22) in diesem trichterförmigen Bereich (31) angeordnet sind oder an diesem angebracht sind.

4. Brustpumpe nach einem der vorhergehenden Ansprüche, wobei die Elektroden (22, 23, 43, 44, 45) auf der Brusthaube zwischen 1 Uhr und 11 Uhr angebracht sind, vorzugsweise im Bereich zwischen 1 Uhr und 4 Uhr und/oder im Bereich zwischen 8 Uhr und 11 Uhr.

5. Brustpumpe nach einem der vorhergehenden Ansprüche, wobei die TENS Einheit (11) einen kontinuierlichen und/oder wischende (sweeped) und/oder gepulste Stimulation liefert, vorzugsweise im Bereich unterhalb von 200 µA, vorzugsweise unterhalb von 100 µA und noch bevorzugter im Bereich von 10 bis 30 µA.

6. Brustpumpe nach Anspruch 5, wobei die TENS Einheit (11) eine gepulste Stimulation in einem Frequenzbereich von 0 bis 250 Hz liefert, vorzugsweise in einem Frequenzbereich von 10 bis 250 Hz, wobei die Pulslänge vorzugsweise im Bereich von 10 bis 1000 µs, noch bevorzugter im Bereich von 50 bis 200 µs liegt.

7. Brustpumpe nach einem der vorhergehenden Ansprüche, wobei die Brustpumpe einen Vakuumschlauch (20) für diese Kommunikation zwischen der Druckquelle (10) und der Brusthaube (30, 31) aufweist und wobei die elektrische Leitung (21) mindestens über einen Teil der Länge des Vakuumschlauchs (20) mit diesem Vakuumschlauch (20) verbunden ist.

8. Brustpumpe nach einem der vorhergehenden Ansprüche, wobei sie Kontrollmittel (10', 11') aufweist, um einen Betrieb der Vakuumquelle (10) und der TENS Einheit (11) zur selben Zeit zu verhindern.

9. Brustpumpe nach einem der vorhergehenden Ansprüche, wobei die TENS Einheit (11) programmiert ist, um Interferenzstromtherapie (IFC) zu liefern.

## Revendications

1. Tire-lait comprenant une téterelle (30, 31) ayant une partie (31) à l'intérieur de laquelle le sein d'une femme est reçu pour l'expression du lait et une source de pression (10) en communication avec ladite téterelle (30, 31), **caractérisé en ce que** le tire-lait a des moyens (11, 21, 22, 23, 43, 44, 45) pour la stimulation nerveuse électrique transcutanée (TENS) de la femme, dans lequel lesdits moyens (11, 21, 22, 23, 43, 44, 45) de TENS comprennent une unité de TENS (11) pour générer des impulsions électriques, des électrodes (22, 23, 43, 44, 45) pour appliquer lesdites impulsions électriques sur ladite femme et un fil électrique (21) pour transmettre lesdites impulsions électriques de ladite unité de TENS (11) aux électrodes (22, 23, 43, 44, 45) et dans lequel les électrodes (22) sont intégrées dans la téterelle (30, 31) ou fixées à celle-ci.

2. Tire-lait selon la revendication 1, dans lequel l'unité de TENS (11) est fixée à ladite source de pression (10) ou intégrée dans celle-ci.

3. Tire-lait selon la revendication 1, dans lequel la téterelle (30, 31) comprend une partie en forme d'entonnoir (31) et dans lequel les électrodes (22) sont agencées dans cette partie en forme d'entonnoir (31) ou fixées à celle-ci.

4. Tire-lait selon l'une quelconque des revendications précédentes, dans lequel les électrodes (22, 23, 43, 44, 45) sont positionnées sur la téterelle entre 1 heure et 11 heures, de préférence dans la région entre 1 heure et 4 heures et/ou dans la région entre 8 heures et 11 heures.

5. Tire-lait selon l'une quelconque des revendications précédentes, dans lequel l'unité de TENS (11) fournit une stimulation continue et/ou balayée (sweeped) et/ou pulsée, de préférence avec un courant dans une plage inférieure à 200 µA, de préférence inférieure à 100 µA, de façon davantage préférée encore dans la plage de 10-30 µA.

6. Tire-lait selon la revendication 5, dans lequel l'unité de TENS (11) fournit une stimulation pulsée dans une gamme de fréquences de 0-250 Hz, de préférence dans une gamme de fréquences de 10-250 Hz, dans lequel la longueur d'impulsion est de préférence dans la plage de 10-1000 µs, de façon davantage préférée encore dans la plage de 50-200 µs.

7. Tire-lait selon l'une quelconque des revendications précédentes, dans lequel le tire-lait comprend un tube de vide (20) pour ladite communication entre la source de pression (10) et la téterelle (30, 31) et dans lequel le fil électrique (21) est relié audit tube de vide (20) au moins le long d'une partie de la longueur du tube de vide (20).

8. Tire-lait selon l'une quelconque des revendications précédentes, dans lequel il comprend des moyens de commande (10', 11') pour empêcher l'utilisation de la source de vide (10) et de l'unité de TENS (11) en même temps.

9. Tire-lait selon l'une quelconque des revendications précédentes, dans lequel l'unité de TENS (11) est programmée pour fournir une thérapie à traitement de courant interférentiel (IFC).
